# EUROPEAN PATENT APPLICATION

(11) **EP 3 241 497 A1**
(43) Date of publication of application: **08.11.2017**
(21) Application number: 15875028.1
(22) Date of filing: 27.11.2015
(51) Int. Cl.: A61B 6/03

(54) **COMPUTED-TOMOGRAPHY METHOD AND DEVICE**

(30) Priority: 30.12.2014 CN 201410857172
(71) Applicant: Shanghai UEG Medical Devices Co., Ltd., Shanghai 201318 (CN)
(72) Inventor: XI, Yan, Shanghai 201318 (CN); YAO, Jun, Shanghai 201318 (CN); LI, Minxu, Shanghai 201318 (CN)
(74) Representative: V.O.
(86) International application number: PCT/CN2015/095734
(87) International publication number: WO 2016/107349

(57) **Abstract**

An imaging method comprises the steps of: putting an object in a detection region, and biasing a detector (1-8) relative to the object; moving an imaging system along a longitudinal Z axis, enabling a ray source (1-7) and the detector (1-8) to synchronously perform circular movement around the object, performing scanning and data collection, and supplementing the data; and reconstructing the collected data to obtain a complete object image. The imaging method combines detector biasing and spiral scanning, solves the problem that an image splicing method used in conventional CT imaging generates artifacts, reduces the usage area of the detector, and reduces system cost.

## Description

### CROSS-REFERENCE TO RELATED APPLICATIONS

This application claims the benefit of priority to Chinese Patent Application No. 201410857172.0, filed on December 30, 2014, and entitled "COMPUTED-TOMOGRAPHY METHOD AND DEVICE", the entire disclosure of which is incorporated herein by reference.

### TECHNICAL FIELD

The present disclosure generally relates to an imaging method, and more particularly, to a Computed-Tomography (CT) method and a device for implementing the method.

### BACKGROUND

Since the invention of the first CT prototype in 1971, CT imaging technology has played an important role in modem medical diagnosis. A CT imaging device having large imaging view is an urgent need for clinical application. However, due to high cost of CT detectors, the size of the CT detectors significantly affects manufacturing cost of CT devices. A CT imaging device configured with a large-size detector may have relatively high manufacturing cost.

To obtain a larger CT imaging coverage volume, helical scanning is an ideal way to increase the imaging view. According to search results of an academic paper database, a German scientist Willi Kalender developed the first helical scanning CT device in 1989, which can effectively improve the CT imaging device in a coverage area in a Z-axis direction (a moving direction of bed during a scanning process of a patient), to achieve continuous CT scan of a "long" object. According to related patent search, existing patents, such as 200410026596.9, describe using helical scanning to improve the coverage area of the CT imaging device in the Z-axis direction (the Z-axis is a coordinate axis facing a plane where a CT tomographic image is located). However, the helical scanning method is limited to improve the imaging view in the Z-axis direction. The size of a required detector can only be reduced in the Z-axis direction, and it cannot significantly reduce the size of the detector required in the CT imaging device under the precondition that the imaging view retains unchanged.

Currently, on the market, some cone-shaped beam CT device manufacturers have expanded a coverage area in the Z-axis direction of the cone-shaped beam CT device by moving a scanning device (mainly including an X-ray tube and a detector) overall. This method includes: fixing the imaging system at a certain height, performing CT scanning and image reconstruction to obtain three-dimensional volume data within an imaging range, raising the scanning device of the imaging system (including an X-ray source, the detector, etc.) to another height to perform another CT imaging, so as to obtain three-dimensional volume data at the new height; and finally, splicing the three-dimensional volume data obtained by the two imaging processes to get complete three-dimensional volume data within long Z-axis coverage. An obvious disadvantage of the method lies in that a splicing position of the two set of three-dimensional volume leaves obvious image artifact which is detrimental to doctor's identification and diagnosis. Besides, the CT scanning is performed at two locations respectively, which requires additional movement of the scanning device. As a result, the overall scan time period is increased, and it is prone to cause unnecessary movement artifacts in the image.

### SUMMARY

An object of embodiments of the present disclosure is to provide an imaging method which achieves large imaging view by using a computer to photograph a body section.

Another object of embodiments of the present disclosure is to provide an imaging method which significantly improves efficiency of cone-shaped beam CT on the use of a detector to reduce cost of a CT device under the precondition that an imaging view and imaging quality retain unchanged.

Another object of embodiments of the present disclosure is to provide an imaging method which significantly reduces a scanning time of an object with a long Z-axis size.

Another object of embodiments of the present disclosure is to provide an imaging method which significantly reduces impact on identification and diagnosis caused by artifacts of image splicing.

Another object of embodiments of the present disclosure is to provide an imaging device to implement various image methods.

In an embodiment of the present disclosure, an imaging method is provided, including: putting an object in a detection region, and biasing a detector relative to the object, to make a portion of data of scanning the object with a ray source be obtained by the detector; moving an imaging system which consists of the ray source and the detector along a longitudinal Z axis, enabling the ray source and the detector to synchronously perform circular movement around the object, and performing scanning and data collection; and reconstructing collected data to obtain a complete object image.

In the above embodiments, the ray source and the detector are biased relative to the object. To image objects with different specifications or when a specification of the detector changes, whether the object is located at a central imaging region is detected.

In another embodiment of the present disclosure, an imaging method is provided, including:
putting an object in a detection region and biasing a detector relative to the object, to make a portion of data of scanning the object with a ray source be obtained by the detector;
according to requirements of two-dimensional projection imaging range, repeating the following steps to adjust an imaging range and performing image splicing, so as to realize target imaging region positioning;
where the following steps include:
   i) first, adjusting a position of the detector and obtaining, from the detector, data of a first projection of the object by the ray source, moving the detector in a horizontal direction or moving an imaging system (including the ray source and the detector) in a vertical direction to obtain data of a second projection of the object by the ray source to supplement data that was not acquired in the first projection; combining the data of the first projection with the data of the second projection; and if a first desired projection image is not obtained, continuing repeating the step i) to collect more projection images at different positions until the first desired projection image is obtained;
   ii) afterwards, rotating the ray source together with a detecting component by 90 degrees relative to the object; and
   iii) afterwards, adjusting the position of the detector according to the step i) and obtaining, from the detector, data of a third projection of the object by the ray source, moving the detector in the horizontal direction or moving the imaging system (including the ray source and the detector) in the vertical direction to obtain data of a fourth projection of the object by the ray source to supplement data that was not acquired in the third projection; combining the data of the third projection with the data of the fourth projection; and if a second desired projection image is not obtained, continuing repeating the step iii) to meet requirements of target imaging region positioning under a current degree;
moving the object along a longitudinal Z axis, enabling the ray source and the detector to synchronously perform circular movement around the object, and performing scanning and data collection; and
reconstructing the collected data to obtain a complete object image.

In some embodiments, a maximum rate of the object moving along the longitudinal Z axis is p/t, where p is a height of the detector in the Z-axis direction, and t is a time period for the ray source and the detector to rotate 360 degrees.

In some embodiments, a line defined by a focus of the ray source and a center of the ray source and the detecting component is intersected with the detector. The detecting component includes the detector and a sliding rail structure, and the detector slides on the sliding rail structure.

In some embodiments, the ray source and the detector may rotate at least 360 degrees around the object.

In some embodiments, the detector may be a flat panel detector.

In some embodiments, the object includes a living body, such as a person, a wild animal or a livestock. The wild animal is an animal that is not artificially acclimated in a natural state. A livestock is an animal that is artificially fed for providing food sources, such as a dog, a cat, a mouse, a hamster, a pig, a rabbit, a cow, a buffalo, a bull, a sheep, a goat, a goose or a chook. In some embodiments, the living body may be a mammal, such as a person who stands or sits within the detection region.

In an embodiment of the present disclosure, to implement various imaging methods provided in the embodiments of the present disclosure, an imaging device is provided, including: a frame body, configured to move upward or downward; a rotation frame which is flexibly connected with the frame body and includes a sliding rail structure; a data transmission component which is disposed at a joint between the frame body and the rotation frame and connected with a power line and a data line respectively; a ray source disposed on the rotation frame; and a detector which slides on the sliding rail structure.

In some embodiments, the detector may be a flat panel detector.

In some embodiments, the sliding rail structure may include at least one sliding rail, and the detector slides on the at least one sliding rail.

In some embodiments, the joint between the frame body and the rotation frame may be a rotation center, and when the rotation frame rotates, an area covered by the ray source and the detector always includes the rotation center.

Embodiments of the present disclosure may provide following advantages. The methods provided in embodiments of the present disclosure combines flat panel detector biasing with spiral scanning, which solves the problem that an image splicing method used in conventional CT imaging (particularly cone-shaped beam CT imaging) generates artifacts by cone-shaped beam CT covering the longitudinal Z-axis.

Further, by combining detector biasing with spiral scanning, an imaging view in an X-Y plane and an imaging view in the Z-axis direction may be enlarged, and an imaging method with a large view projection is realized by using a flat panel detector with a relatively small area.

When the imaging methods provided in embodiments of the present disclosure are applied to the cone-shaped beam CT imaging, a flat panel detector with a relatively small size (such as 18cm*7cm) can be used to realize large-view imaging. Cost of a whole CT imaging device may be significantly reduced under the precondition that an imaging view retains unchanged.

The imaging methods provided in embodiments of the present disclosure are adapted to living bodies, such as a person who stands or sits within the detection region, which significantly improves adaptability of medical treatment.

In the imaging device provided in embodiments of the present disclosure, a slip ring structure is used at the rotation center, which allows the ray source and the detector to perform continuous rotation scanning imaging. In this way, a scanning time period may be reduced, and generation of potential movement artifacts may be avoided effectively.

In the imaging device provided in embodiments of the present disclosure, the sliding rail structure is used, which is helpful to adjust a horizontal position of the detector to perform full-field imaging to solve a positioning problem caused by detector biasing.

### BRIEF DESCRIPTION OF THE DRAWINGS

Figure 1 schematically illustrates a structural diagram of an imaging device which implements an imaging method according to an embodiment of the present disclosure;
Figure 2 schematically illustrates a structural diagram of a data transmission component shown in Figure 1 according to an embodiment of the present disclosure;
Figure 3 schematically illustrates a structural diagram of a detector shown in Figure 1 according to an embodiment of the present disclosure;
Figure 4 schematically illustrates a CT diagram of imaging data of a first projection of the object by the ray source obtained from the detector according to an embodiment of the present disclosure;
Figure 5 schematically illustrates a CT diagram of imaging data of a second projection of the object by the ray source obtained from the detector according to an embodiment of the present disclosure;
Figure 6 schematically illustrates combining the data of the first projection with the data of the second projection to obtain a complete projection image;
Figure 7 schematically illustrates a diagram of performing bias scanning and imaging to an object according to an embodiment of the present disclosure;
Figure 8 schematically illustrates a diagram of supplementing missing data for one-side bias scanning when an imaging method provided in an embodiment of the present disclosure is employed;
Figure 9 schematically illustrates a trajectory diagram of scanning an object by using an imaging method provided in an embodiment of the present disclosure;
Figure 10 schematically illustrates a projection image which is generated by imaging data collected by scanning an object using an imaging method provided in an embodiment of the present disclosure; and
Figure 11 schematically illustrates a flow chart of an imaging method according to an embodiment of the present disclosure.

### DETAILED DESCRIPTION

Technical solutions of the present disclosure are described in detail in conjunction with accompanying figures. Embodiments of the present disclosure are used to describe but not limit the technical solutions of the present disclosure. Although the present disclosure is described in detail with reference to preferred embodiments, those skilled in the art should understand that the technical solutions of the present disclosure can be modified or equivalently replaced without departing from the spirit and scope of the present disclosure, which should be contained in the scope of claims of the present disclosure.

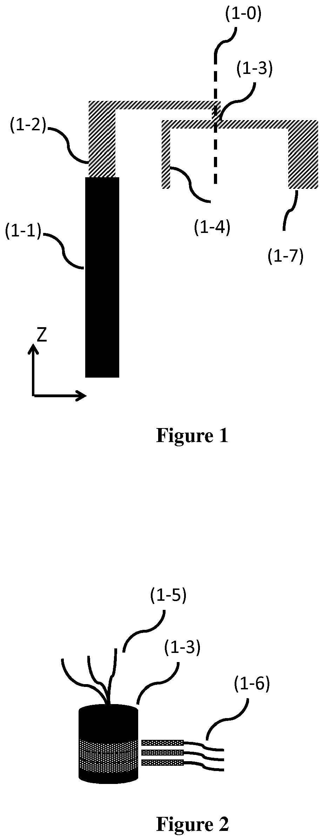

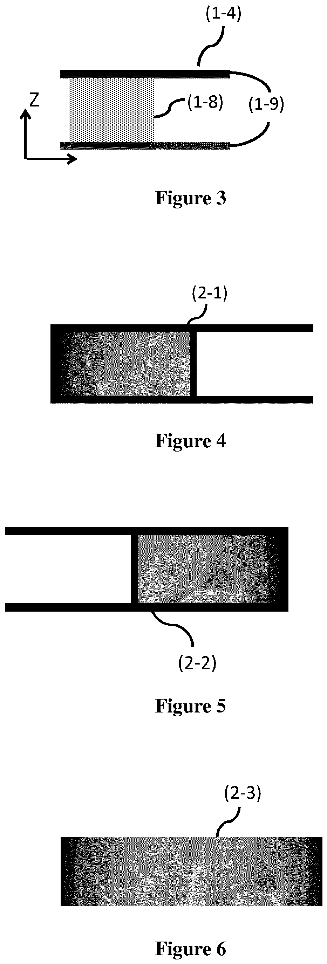

Figure 1 schematically illustrates a structural diagram of an imaging device which implements an imaging method according to an embodiment of the present disclosure, Figure 2 schematically illustrates a structural diagram of a data transmission component shown in Figure 1 according to an embodiment of the present disclosure, and Figure 3 schematically illustrates a structural diagram of a detector shown in Figure 1 according to an embodiment of the present disclosure. Referring to Figures 1, 2 and 3, a CT scanning system (for example, a cone-shaped beam CT) is designed to include an imaging part (1-2) and a pillar part (1-1). The imaging part (1-2) is a sliding structure in the pillar part (1-1), which can move upward and downward in a vertical direction.

A power line (1-5) and a data line (1-6) are connected via a slip ring system (1-3) instead of a conventional wire, so that a detecting component (1-4) and a ray source (1-7) can continuously rotate around an object to prevent wire entanglement.

The detecting component (1-4) includes two parallel guide rails (1-9), and a detector (1-8) slides on the two parallel guide rails (1-9). In some embodiments, the guide rails may be a sliding rail structure which facilitates adjusting a horizontal position of the detector for full-field imaging, to solve a positioning problem caused by biasing the detector. An area of the detector (1-8) may be smaller than an area between the two parallel guide rails (1-9). In some embodiments, the detector (1-8) may be a cone-shaped beam CT flat panel detector with a length of 18cm and a width of 7cm. During a CT scanning process, the imaging part moves, and the detector and the ray source rotate around the object, to realize spiral scanning.

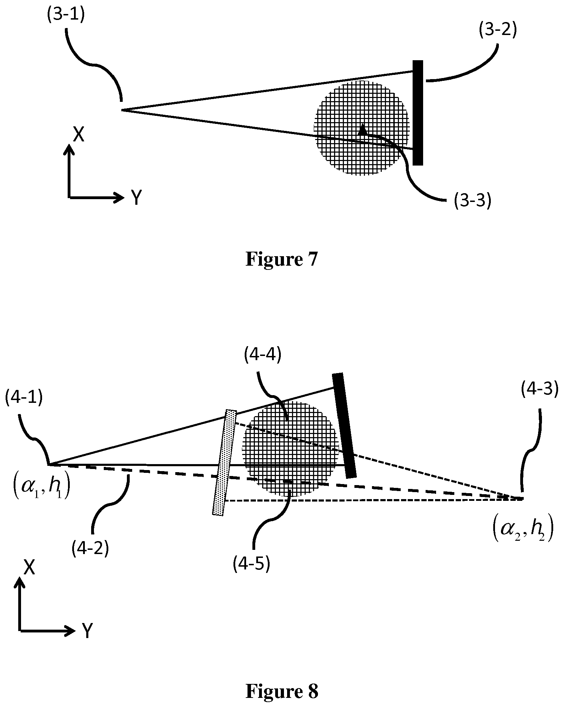

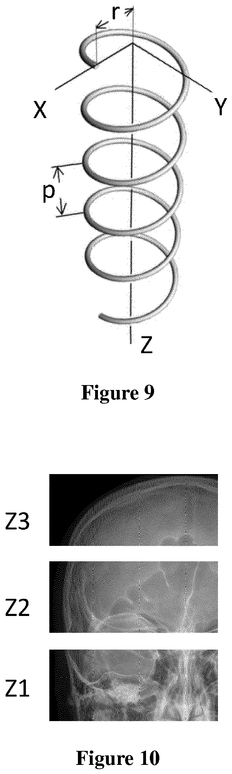

Figure 7 schematically illustrates a diagram of performing bias scanning and imaging to an object according to an embodiment of the present disclosure. The detector of the CT imaging system is placed biased relative to the object, and a line defined by a focus (3-1) of the ray source and a center (3-3) of the ray source and the detecting component is intersected with a flat panel detector (3-2). Referring to Figures 1, 3 and 7, the detector (1-8) is fixed on a guide rail in an X direction, and the flat panel detector (1-8) is electrically controlled to move along the guide rails (1-9). The detector (1-8) is biased, that is, the detector is biased to one side of the X direction, where a circular shaded portion is a target region (2-4) to be performed with image reconstruction. During a CT scanning process, the imaging part (1-2) moves along a longitudinal Z axis. Meanwhile, the ray source (1-7) and the detecting component (1-4) perform circular movement around the object. X rays are exposed and data is collected, where a scanning trajectory is shown in Figure 9. In some embodiments, the object may be a person who stands or sits within the detection region.

A maximum rate of the object moving along the longitudinal Z axis is p/t, where p is a height of the detector in the Z-axis direction, and t is a time period for the ray source and the detector to rotate 360 degrees. For example, if the height of the detector in the Z-axis direction is 7cm, and the time period for CT scanning of a circle is 10 seconds, a movement rate of the object during the CT scanning process is 0.7cm per second. A rotation rate of the ray source and the detector is 36 degrees per second.

Referring to Figure 7, the ray source emits X rays (3-1), and the detector detects X-ray signals (3-2) and performs continuous circular movement around the object to be scanned, until a moving distance of the imaging part of the CT scanning system completely covers the object to be scanned and the ray source rotates at least 360 degrees. A rotation center of the circular movement performed by the ray source and the detector can be referred to the point (1-0) in Figure 1 or the point (3-3) in Figure 7. A data collection system consisting of the ray source and the detector does not need to completely cover the whole plane to be imaged. Instead, it only needs to ensure that, during the rotation along the rotation center, an area covered by the ray source and the detector always includes the rotation center.

Figure 9 schematically illustrates a trajectory diagram of CT scanning using an imaging method provided in an embodiment of the present disclosure. At a same angle of the ray source, with the movement of the object along the longitudinal Z axis, different portions (Z1, Z2 and Z3) of the object are scanned. Projections data is collected by the detector and further imaged, as shown in Figure 10.

To image objects with different specifications or perform imaging using detectors with different specifications, whether the object is located at a central imaging region is detected. Figure 4 schematically illustrates features of an image formed in bias spiral CT. As an area of the detector is relatively small, imaging of one time cannot cover a horizontal structure of a complete object but only covers a first partial feature (2-1). The flat panel detector slides along the slide rails to perform another imaging, to obtain a second partial feature (2-2) (referring to Figure 5). The second partial feature can supplement content that was not presented in the first partial feature. The results in the two imaging processes are combined to obtain a complete head projection image (2-3) (referring to Figure 6). A projection image with the ray source at a direction of 90° may be obtained in a similar way, to determine whether the object is located in the central imaging plane.

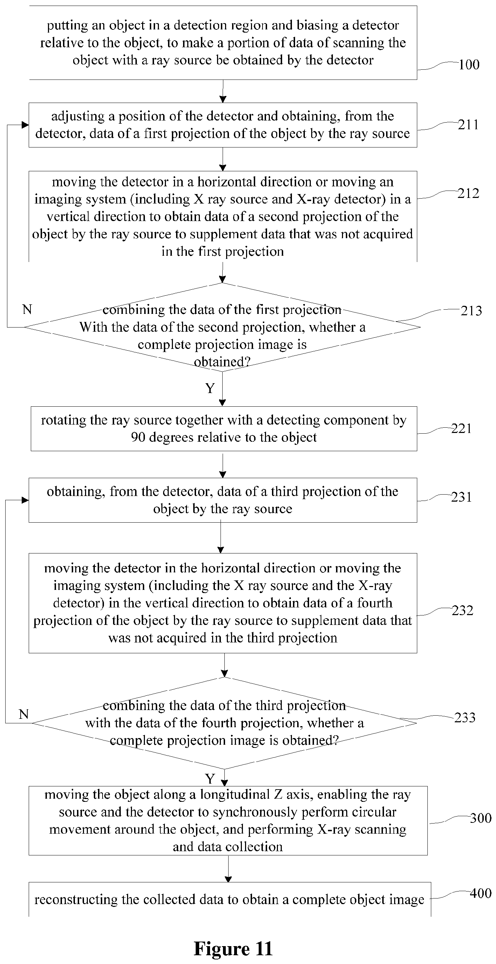

Figure 11 schematically illustrates a flow chart of an imaging method according to an embodiment of the present disclosure. The imaging method includes:
step 10, putting an object in a detection region and biasing a detector relative to the object, to make a portion of data of scanning the object with a ray source be obtained by the detector;
according to requirements of two-dimensional projection imaging range, adjusting an imaging range and performing image splicing, so as to realize target imaging region positioning;
where the above step includes:
   step 211, adjusting a position of the detector and obtaining, from the detector, data of a first projection of the object by the ray source; step 212, moving the detector in a horizontal direction or moving an imaging system (including X ray source and X-ray detector) in a vertical direction to obtain data of a second projection of the object by the ray source to supplement data that was not acquired in the first projection; step 213, combining the data of the first projection with the data of the second projection to obtain a complete projection image; and if a first desired projection image is not obtained, continuing repeating the steps 221 to 223 to collect more projection images at different positions until the first desired projection image is obtained;
   afterwards, step 221, rotating the ray source together with a detecting component by 90 degrees relative to the object; and
   afterwards, step 231, obtaining, from the detector, data of a third projection of the object by the ray source; step 232, moving the detector in the horizontal direction or moving the imaging system (including the X ray source and the X-ray detector) in the vertical direction to obtain data of a fourth projection of the object by the ray source to supplement data that was not acquired in the third projection; S233, combining the data of the third projection with the data of the fourth projection to obtain a complete projection image at a position where the ray source is rotated by 90 degrees; and if a second desired projection image is not obtained, continuing repeating the steps 231 to 233 to meet requirements of target imaging region positioning under a current degree;
   step 30, moving the object along a longitudinal Z axis, enabling the ray source and the detector to synchronously perform circular movement around the object, and performing X-ray scanning and data collection; and
   step 40, reconstructing the collected data to obtain a complete object image.

During image reconstruction, data needs to be supplemented.

In the embodiments, as the detector has a relatively small size, projection imaging at one angle cannot cover a complete target imaging region. Thus, three-dimensional volume data reconstruction strategy based on contralateral image supplement is employed. Figure 8 schematically illustrates a diagram of supplementing missing data for one-side bias scanning when the imaging method provided in the embodiment of the present disclosure is employed. Referring to Figure 8, when the ray source moves to an angle of *α*₁ and a position of h₁ (4-1), X ray signals can be detected by the detector only at a partial region (4-4) in Figure 4, while there is no detector for a plane indicated by a region (4-5) in Figure 4 to perform data collection. To supplement missing signals of the region (4-5), the ray source should rotate to other positions to enable the detector to collect projection data for supplement. Take missing data supplement shown as dotted line (4-2) in Figure 4 as an example. When the ray source is located at an original position (*α*₁, *h*₁), as a horizontal size of the detector is not great enough to cover the whole imaging plane, projection data of the ray source at the original position (*α*₁, *h*₁) is supplemented by projection data of the ray source at a position *α*₂ (4-3), which is shown as dotted lines in Figure 4. The dotted line (4-2) in Figure 4 should be intersected with the ray source at the position *α*₂ and a plane where the detector corresponding to the ray source at the position *α*₂ is located.

The above procedure can be interpreted as follows. At the angle of *α*₁, projection data of a point *f*(*x,h*) in a region to be reconstructed is not collected by the detector. In this situation, a focus of the ray source at the angle of *α*₁ and the point *f*(*x,h*) in the region to be reconstructed are connected to form a straight line. An angle between the straight line and a line defined by the focus of the ray source at the angle of *α*₁ and a rotation center of the imaging system is Δ*α*. To supplement missing data of the point *f*(*x,h*) at the angle of *α*₁, when the ray source moves to the position *α*₂ (***α*₂ = *α*₁ + 180° ± Δ*α***), measurement values at a position where the straight line is intersected with the detected are used to perform data supplement. When the ray source is taken as the vision, the detector is out of sight at its right view, and the ray source rotates rightward, projection data at the angle of ***α*₂ = *α*₁ + 180° - Δ*α*** is obtained; when the ray source is taken as the vision, the detector is out of sight at its right view, and the ray source rotates leftward, projection data at the angle of ***α*₂** = ***α*₁ + 180° + Δ*α*** is obtained; when the ray source is taken as the vision, the detector is out of sight at its left view, and the ray source rotates leftward, projection data at the angle of ***α*₂ = *α*₁ + 180° - Δ*α*** is obtained; when the ray source is taken as the vision, the detector is out of sight at its left view, and the ray source rotates rightward, projection data at the angle of ***α*₂ = *α*₁ + 180° + Δ*α*** is obtained

The supplemented data may be used for filtering. With NVIDIA's graphics computing card and CUDA parallel computing technology, in accordance with the classic filter back projection reconstruction algorithm, three-dimensional volume data is reconstructed. The three-dimensional volume data reconstruction includes two parts, image filtering and back projection. In the image filtering, complete projection data obtained by the method described in the above supplement strategy is used to filter; and in the back projection step, only the filtered data directly measured at each angle is used for back projection, while the data supplemented by the supplement strategy is not subjected to the back projection.

Currently, although some cone-shaped beam CT enterprise has used a translation method of a ray source and a detector to perform CT scanning on two positions and then perform image slicing to achieve long Z-axis coverage, the method requires additional movement of a scanning device. As a result, the overall scan time period is increased, and it is prone to cause unnecessary movement artifacts in the image.

Some cone-shaped beam CT enterprise employs a detector biasing method to expand an imaging view. However, the method only expands the imaging view on an X-Y plane but cannot expand the imaging view in a Z direction. Embodiments of the present disclosure provide methods which combine detector biasing with spiral scanning and expand imaging views on both an X-Y plane and a Z direction. Further, a large view projection imaging can be realized using a flat panel detector with a relatively small area.

## Claims

1. An imaging method, comprising:
putting an object in a detection region, and biasing a detector relative to the object, to make a portion of data of scanning the object with a ray source be obtained by the detector;
moving an imaging system which consists of the ray source and the detector along a longitudinal Z axis, enabling the ray source and the detector to synchronously perform circular movement around the object, and performing scanning and data collection; and
reconstructing collected data to obtain a complete object image.

2. The imaging method according to claim 1, further comprising: when the collected data is constructed, supplementing the collected data, wherein supplementing the collected data comprises:
at an angle of *α*₁, projection data of a point *f*(*x,h*) in a region to be reconstructed being not collected by the detector; a focus of the ray source at the angle of *α*₁ and the point *f*(*x,h*) in the region to be reconstructed being connected to form a straight line, where an angle between the straight line and a line defined by the focus of the ray source at the angle of *α*₁ and a rotation center of an imaging system is Δ*α*; and to supplement missing data of the point *f*(*x,h*) at the angle of *α*₁, when the ray source moves to a position *α*₂, using measurement values at a position where the straight line is intersected with the detected to perform data supplement, where ***α*₂ = *α*₁ + 180° ± Δ*α*,** and the imaging system comprises the ray source and the detector.

3. The imaging method according to claim 1, wherein a maximum rate of the object moving along the longitudinal Z axis is p/t, where p is a height of the detector in the Z-axis direction, and t is a time period for the ray source and the detector to rotate 360 degrees.

4. The imaging method according to claim 1, wherein the ray source and the detector rotate at least 360 degrees around the object.

5. The imaging method according to claim 1, wherein a line defined by a focus of the ray source and a center of the ray source and the detecting component is intersected with the detector, and the detecting component comprises the detector.

6. The imaging method according to claim 1, wherein the object is a living body who stands within the detection region.

7. The imaging method according to claim 1, wherein the object is a person who stands or sits within the detection region.

8. An imaging method, comprising:
putting an object in a detection region and biasing a detector relative to the object, to make a portion of data of scanning the object with a ray source be obtained by the detector;
according to requirements of two-dimensional projection imaging range, repeating the following steps to adjust an imaging range and performing image splicing, so as to realize target imaging region positioning, where the following steps comprise:
i) first, adjusting a position of the detector and obtaining, from the detector, data of a first projection of the object by the ray source, moving the detector in a horizontal direction or moving an imaging system (comprising the ray source and the detector) in a vertical direction to obtain data of a second projection of the object by the ray source to supplement data that was not acquired in the first projection; combining the data of the first projection with the data of the second projection; and if a first desired projection image is not obtained, continuing repeating the step i) to collect more projection images at different positions until the first desired projection image is obtained;
ii) afterwards, rotating the ray source together with a detecting component by 90 degrees relative to the object; and
iii) afterwards, adjusting the position of the detector according to the step i) and obtaining, from the detector, data of a third projection of the object by the ray source, moving the detector in the horizontal direction or moving the imaging system (comprising the ray source and the detector) in the vertical direction to obtain data of a fourth projection of the object by the ray source to supplement data that was not acquired in the third projection; combining the data of the third projection with the data of the fourth projection; and if a second desired projection image is not obtained, continuing repeating the step iii) to meet requirements of target imaging region positioning under a current degree;
moving the object along a longitudinal Z axis, enabling the ray source and the detector to synchronously perform circular movement around the object, and performing scanning and data collection; and
reconstructing the collected data to obtain a complete object image.

9. The imaging method according to claim 8, further comprising: when the collected data is constructed, supplementing the collected data, wherein supplementing the collected data comprises:
at an angle of *α*₁, projection data of a point *f*(*x,h*) in a region to be reconstructed being not collected by the detector; a focus of the ray source at the angle of *α*₁ and the point *f*(*x,h*) in the region to be reconstructed being connected to form a straight line, where an angle between the straight line and a line defined by the focus of the ray source at the angle of *α*₁ and a rotation center of an imaging system is Δ*α*; to supplement missing data of the point *f*(*x,h*) at the angle of *α*₁, when the ray source moves to a position *α*₂, using measurement values at a position where the straight line is intersected with the detected to perform data supplement, where ***α*₂ = *α*₁ + 180° ± Δ*α*,** and the imaging system comprises the ray source and the detector.

10. The imaging method according to claim 8, wherein a maximum rate of the object moving along the longitudinal Z axis is p/t, where p is a height of the detector in the Z-axis direction, and t is a time period for the ray source and the detector to rotate 360 degrees.

11. The imaging method according to claim 8, wherein the ray source and the detector rotate at least 360 degrees around the object.

12. The imaging method according to claim 8, wherein a line defined by a focus of the ray source and a center of the ray source and the detecting component is intersected with the detector, and the detecting component comprises the detector.

13. The imaging method according to claim 8, wherein the object is a living body who stands within the detection region.

14. The imaging method according to claim 8, wherein the object is a person who stands or sits within the detection region.

15. The imaging method according to any one of claims 1 to 14, wherein the detector is a flat panel detector.

16. An imaging device configured to implement the imaging method according to any one of claims 1 to 14, comprising:
a frame body, configured to move upward or downward;
a rotation frame which is flexibly connected with the frame body and comprises a sliding rail structure;
a data transmission component which is disposed at a joint between the frame body and the rotation frame and connected with a power line and a data line respectively;
a ray source disposed on the rotation frame; and
a detector which slides on the sliding rail structure.

17. The imaging device according to claim 16, wherein the sliding rail structure comprises at least one sliding rail.

18. The imaging method according to claim 16, wherein the joint between the frame body and the rotation frame is a rotation center, and when the rotation frame rotates, an area covered by the ray source and the detector always surrounds the rotation center.
